(19) Europäisches Patentamt / European Patent Office / Office européen des brevets

(11) **EP 1 126 289 B1**

(12) **EUROPEAN PATENT SPECIFICATION**

(45) Date of publication and mention
of the grant of the patent:
**13.11.2013 Bulletin 2013/46**

(51) Int Cl.:
***G01T 1/29*** *(2006.01)*

(21) Application number: **01102319.9**

(22) Date of filing: **01.02.2001**

(54) **Image acquisition method and apparatus**

Bildaufnahmeverfahren und -apparat

Procédé et appareil pour l'acquisition d'images

(84) Designated Contracting States:
**DE FR GB NL**

(30) Priority: **04.02.2000 JP 2000028161**
**04.02.2000 JP 2000028207**

(43) Date of publication of application:
**22.08.2001 Bulletin 2001/34**

(73) Proprietor: **CANON KABUSHIKI KAISHA**
**Tokyo (JP)**

(72) Inventor: **Inoue, Hitoshi**
**Ohta-ku,**
**Tokyo (JP)**

(74) Representative: **TBK**
**Bavariaring 4-6**
**80336 München (DE)**

(56) References cited:
**EP-A- 0 792 060     EP-A- 0 962 888**
**JP-A- 11 113 889     US-A- 4 317 179**
**US-A- 5 666 395     US-B1- 6 201 249**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to an image acquisition technique and, more particularly, to an image acquisition apparatus for acquiring an image corresponding to an X-ray transmission distribution obtained on an object.

BACKGROUND OF THE INVENTION

With the recent trend toward the digitization of medical X-ray images, X-ray intensity spatial distributions can be acquired as digital images. For example, currently available schemes include a scheme of forming a latent image on a photostimulable phosphor by using X-ray energy and acquiring an image from a laser pumping light distribution, a scheme of converting an X-ray intensity distribution into a light intensity distribution (fluorescence), directly converting the distribution into an electrical signal by using a surface center having a plurality of pixels, and converting the signal into a digital image, and a scheme of directly converting an X-ray intensity distribution into a charge distribution.

The digitization of X-ray images has the  following merits:

• Storage and transfer can be efficiently performed.
• Optimal images can be easily formed by digital image processing (a recovery from a failure in imaging operation can be made).
• Efficient diagnosis can be carried out.
• A reduction in the cost of diagnosis can be attained.

The problem of scattered X-rays produced when X-rays pass through an object has not been satisfactorily solved. To reduce the influences of scattered X-rays and obtain a high-contrast image, an optimal means is to use a scattered ray removing grid having many lead plates arranged in the same direction as in a conventional scheme using silver-halide films.

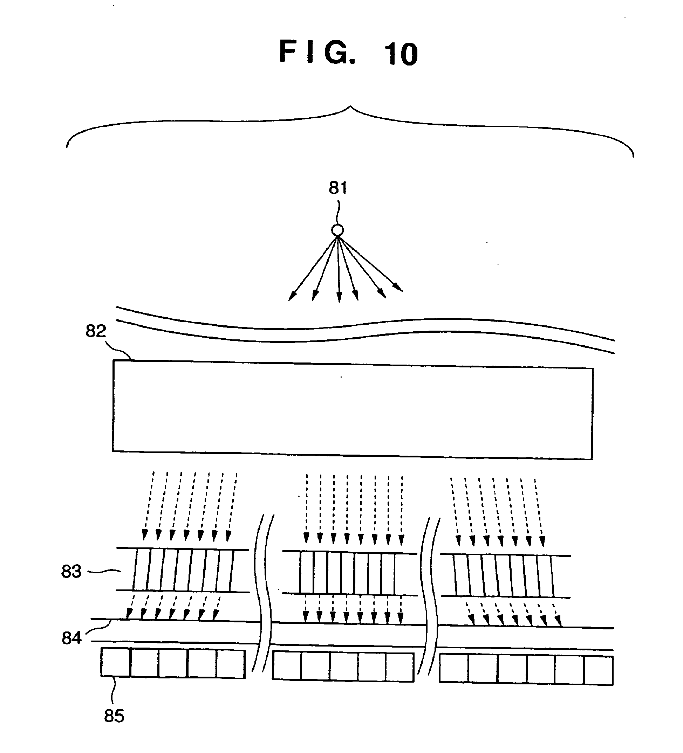

Fig. 10 is a schematic sectional view of a structure using a grid. Reference numeral 81 denotes a point (X-ray focal point) of an X-ray tube from which X-rays are generated; 82, an object; 83, a grid; 84, an energy conversion unit for converting an X-ray intensity distribution into a light intensity or charge amount; and 85, a sensor unit for spatially sampling the distribution. Scattered X-rays reflected by the object reach the grid 83 as well as X-rays directly  emitted from the X-ray tube. Most of the scattered X-rays are cut by the grid 83 made up of lead members facing the X-ray focal point 81.

A drawback of the grid 83 is that it partly cuts direct X-rays while it cuts scattered X-rays. This cutting pattern corresponds to the arrangement of the lead members of the grid, and the image generally suffers a stripe pattern. An X-ray image has evolved into (1) an image formed by a film-screen system (analog image) - (2) a digital image formed by reading a latent image formed by a photostimulable phosphor by laser scanning - (3) an image formed by direct sampling (flat panel sensor) the two-dimensional spatial distribution of an X-ray dose in a two-dimensional space. Different measures have therefore been taken against a stripe pattern (grid image) on an image which originates from the lead members used for the grid 83.

In the film-screen system in (1), the following two methods are available, which are used to remove a grid image or prevent interference with observation.

(a) The grid itself is moved during radiation of X-rays to prevent the formation of a grid image while removing scattered rays.
(b) The spatial frequency of the grid stripe pattern is increased to make it difficult for the human  eye to perceive a grid image if it is formed on an image or prevent the grid image from overlapping the frequency component of image information.

The means of moving the grid itself in (a) is effective in all cases of X-ray image acquisition. However, this means is difficult-to use because of an increase in cost due to, for example, a driving system for moving the grid, an increase in apparatus size, the relationship between the driving timing and the X-ray radiation timing, adjustment of the driving speed, and the like.

The means of increasing the spatial frequency of the grid stripe pattern in (b) has its own limit. That is, when the frequency of the grid stripe pattern is set to a high spatial frequency at which no grid image is formed, since the thickness of each lead plate for blocking scattered rays is almost fixed, an area through which direct rays are transmitted narrows, and the use efficiency of the X-ray dose extremely decreases. As a consequence, imaging operation cannot be properly performed.

In the era in which a latent image formed by a photostimulable phosphor was read by laser scanning and digitized, the idea of using anti-aliasing filter before sampling was introduced as a method of removing a grid image. When a latent image formed by a photostimulable phosphor is to be read by laser scanning and digitized, the image is scanned in a one-dimensional direction by a laser to temporarily form a signal form like a video signal, and the signal is sampled on the time axis. The frequency of the grid stripe pattern is increased to a certain degree, and laser scanning is performed in a direction perpendicular to the grid stripe pattern to form the grid stripe pattern into a periodic signal on the video signal. A grid image can be removed by the general idea of using an anti-aliasing filter, i.e., performing sampling on the time axis after low-pass filtering in the state of an analog signal as this video signal. A similar method is disclosed in Japanese Patent No. 2507659, in which a grid image and its frequency are obtained by Fourier transformation of an image obtained by preliminary sampling, and a low-pass filter corresponding to the result is selected to remove a grid image.

According to another method, sampling is performed on the time axis at intervals shorter than desired intervals instead of performing analog low-pass filtering to eliminate aliasing of grid stripe pattern information, and the resultant information is separated from image information. Thereafter, digital low-pass filtering is performed, and the resultant image is digitally decimated (sub-sampled), thereby obtaining an image at the desired sampling intervals. Similar methods are disclosed in Japanese Patent No. 2754068 and Japanese Patent Laid-Open No. 8-088765.

In the advanced era in which a digital X-ray image can be obtained by directly sampling (using a flat panel sensor) a two-dimensional spatial distribution of an X-ray dose in (3) in a two-dimensional space, the above anti-aliasing filtering cannot be used. That is, a flat panel sensor is made up of a plurality of semiconductor pixels, and the two-dimensional spatial sampling pitch of the sensor cannot be reduced more than necessary in consideration of technique and cost. The above idea of using an anti-aliasing filter cannot be applied to this method. The method disclosed in Japanese Patent Laid-Open No. 9-75332 is aimed at removing grid stripe pattern information in contrast to the method of obtaining an X-ray image by direct sampling in a two-dimensional space. In this method, the intervals of grid lead members are perfectly matched with the sampling pitch to match areas where direct X-rays are blocked by the grid stripe pattern with the gaps between the pixels, thereby preventing a grid stripe pattern from appearing on an image.

Japanese Patent Laid-Open No. 9-78970 and U.S. Patent No. 5,801,385 disclose methods of setting the grid lead member intervals to be smaller than the sampling pitch and equal to or near the width of the opening of a light-receiving portion of one pixel, thereby reducing the contrast of a grid stripe pattern. In the method disclosed in U.S. Patent No. 5,050,198, a grid image is input and stored under a plurality of conditions. When imaging operation is actually performed by using the grid, the obtained image is divided by a grid image of the stored grid images which corresponds to the condition under which the actual imaging operation is performed, thereby removing the grid image.

In the method disclosed in Japanese Patent Laid-Open No. 9-75332, which corresponds to the above technique of obtaining a digital X-ray image by direct sampling in a two-dimensional space using a flat panel in the two-dimensional space, it is very difficult to perfectly match the grid lead member intervals with the sampling pitch. In the methods disclosed in Japanese Patent Laid-Open No. 9-78970 and U.S. Patent No. 5,801,385, a grid image can be effectively removed by reducing the grid lead member intervals below the sampling pitch to be equal or near the width of the opening of a light-receiving portion of one pixel. However, as the flat panel sensor increases in resolution, and the sampling pitch becomes 0.1 mm or less, the grid lead member intervals are required to be very small; 10 or more grid lead members per mm. If the intervals become so small, since the thickness of each lead plate for blocking scattered rays is almost fixed, the areas through which direct rays pass narrow, and the use efficiency of the X-ray dose becomes extremely low. As a consequence, proper imaging operation cannot be performed.

**Prior art which is related to this field of technology can be found in e.g. document** US 6,201,249 B1 **which is post-published and claiming the priority of** JP9-283606 **disclosing an X-ray imaging system including an X-ray sensor having an imaging section for picking up an X-ray image, and a controller for controlling the X-ray sensor and processing an image supplied by the X-ray sensor. The X-ray sensor has a non-volatile storage device for storing information by which the controller specifies conditions of imaging performed by the imaging section, and the controller has a decision unit for reading out the information stored on the non-volatile storage device and deciding, on the basis of the information, a method of controlling the X-ray sensor and/or content of processing executed by the controller, and in** US 4,317,179 **disclosing a method of and apparatus for processing a radiographic image in a radiographic image copying system in which an original radiograph scanned with the light beam and the image information of the radiograph is read out by a detector which gives an output to be processed and used for reproducing an image on a recording material. The unsharp masking process may be performed together with a gradation process, reduction of image size, smoothing process and the like.**

## SUMMARY OF THE INVENTION

It is an object of the present invention to provide an image acquisition method and apparatus which can acquire an image which prevents a stripe pattern originating from a scattered ray removing grid from interfering with observation when X-rays are radiated by using the scattered ray removing grid.

It is another object of the present invention to provide an image acquisition method and apparatus which can remove a stripe pattern, in an image, which originates from a scattered ray removing grid when X-rays are radiated by using the scattered ray removing grid.

According to an aspect of the present invention, there is provided an image acquisition apparatus and a method according to the respective independent claims. Further details are set forth in the respective dependent claims.

According to the present invention, since whether to remove a stripe pattern originating from the scattered ray removing grid from an image can be selected, an appropriate image can be obtained by removing the stripe pattern originating from the scattered ray removing grid only when required.

Other objects and advantages besides those discussed above shall be apparent to those skilled in the art from the description of a preferred embodiment of the invention which follows. In the description, reference is made to accompanying drawings, which form a part thereof, and which illustrate an example of the invention. Such example, however, is not exhaustive of the various embodiments of the invention, and therefore reference is made to the claims which follow the description for determining the scope of the invention.

BRIEF DESCRIPTION OF THE DRAWINGS

The accompanying drawings, which are incorporated in and constitute a part of the specification, illustrate embodiments of the invention and, together with the description, serve to explain the principles of the invention.

Fig. 1 is a block diagram for explaining the first example useful for understanding the invention;
Figs. 2A and 2B are graphs for explaining spatial spectra in an image;
Fig. 3 is a flow chart for the implementation of the first example useful for understanding the invention by software;
Fig. 4 is a block diagram for explaining the second example useful for understanding the invention;
Fig. 5 is a block diagram for explaining the third embodiment of the present invention;
Fig. 6 is a block diagram for explaining the fourth example useful for understanding the invention;
Figs. 7(1-a) to 7(3-b) are graphs for explaining artifacts originating from a grid stripe pattern;
Figs. 8A and 8B are graphs for explaining beat noise produced when a grid stripe pattern is sampled;
Fig. 9 is a graph showing an example of the spatial frequency range of a grid body; and
Fig. 10 is a view for explaining a scattered ray removing grid.

DETAILED DESCRIPTION OF THE PREFERRED EMBODIMENTS

The examples useful for understanding the invention and embodiment of the present invention will be described below with reference to the accompanying drawings.

(First example useful for understanding the invention)

In an image acquisition apparatus according to the first example useful for understanding the invention, when an acquired image is to be reproduced, the problem in the prior art is solved by setting the intervals of grid lead members such that even if a grid image exists and interferes with an image component to a certain degree, the produced stripes have a frequency which hardly makes an observer have a sense of incongruity.

This example useful for understanding the invention will be described below. In digital images obtained by sampling, image artifacts produced by the grid are classified into the following two categories:

(A) grid images having low spatial frequencies; and

(B) beat images obtained by sampling grid images having low spatial frequencies.

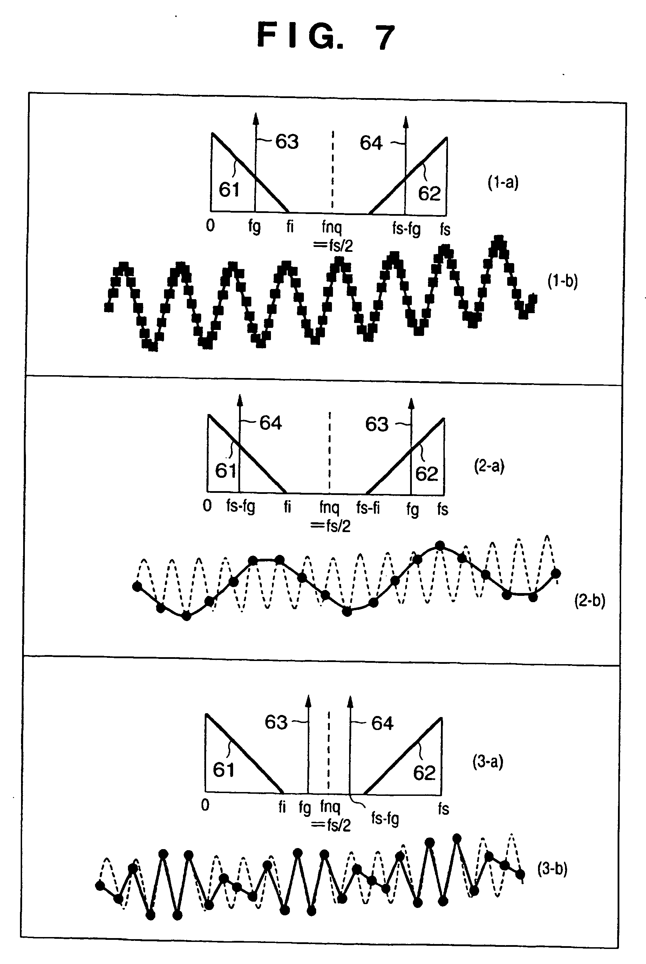

Figs. 7(1-a) to 7(3-b) schematically show the states of artifacts to explain artifacts belonging to categories (A) and (B). For the sake of descriptive convenience, consider only a fundamental wavelength, assuming that the second- and higher-order harmonics of a grid stripe pattern are not resolved.

Fig. 7(1-a) shows the state of an artifact belonging to category (A) in the frequency domain (only the positive region). Reference numeral 61 denotes an image component region, which exhibits a substantially maximum frequency fi; and 62, an image harmonic component mathematically produced by sampling. In this case, a sampling frequency is represented by fs, and Nyquist frequency fnq = fs/2. A line spectrum 63 is a grid stripe pattern component having a frequency fg. As shown in Fig. 7(1-a), since this component overlaps the image component 61 to impair the image quality, and has a low frequency, the observer experiences a sense of incongruity from the stripe pattern.

Fig. 7(1-b) one-dimensionally shows how sampling is performed. In Fig. (1-b), "●" (bullet) indicates a sampling point.

It is obvious from the above description that the frequency fg of the grid stripe pattern and the frequency fi (fi < fs/2) of the image must satisfy:

$$fg > fi \qquad \ldots (1)$$

Fig. 7(2-a) shows the state of another artifact belonging to category (A) in the frequency domain (only in the positive region). A region 61 is an image component region and exhibits the maximum frequency fi. Since the frequency fg of the grid is higher than the Nyquist frequency fnq that is 1/2 the sampling frequency fs, a frequency component 64 of fs - fg, which is aliasing, appears. In the case shown in Fig. 7(2-a), the frequency component 64 as aliasing overlaps the image component 61 and impairs the image quality, and hence a stripe pattern gives the observer a sense of incongruity. Fig. 7(2-b) one-dimensionally shows how sampling is performed. In Fig. (2-b), "●" (bullet) indicates a sampling point, and the dashed line indicates a signal form before sampling. That is, to prevent impairment of the image component, the frequency fg of the grid stripe pattern and the frequency fi (fi < fs/2) of the image must satisfy:

$$fs - fg > fi \qquad \ldots (2)$$

According to inequalities (1) and (2), the frequency fg of the grid stripe pattern must satisfy the following inequality, in relation to the maximum frequency fi of the image and the sampling frequency fs:

$$fi < fg < fs - fi \quad (fi < fs/2) \qquad \ldots (3)$$

In this case as well, an artifact belonging to category (B) may be produced.

Fig. 7(3-a) shows a state of an artifact when the grid stripe pattern frequency is set in the spatial frequency region which satisfies inequality (3). Fig. 7(3-b) one-dimensionally shows how sampling is performed. In Fig. (3-b), "●" (bullet) indicates a sampling point, and the dashed line indicates a signal form before sampling.

Referring to Fig. 7(3-a), it seems that since an image frequency component 61 does not overlap a grid image component 63, the image is not impaired, and the observer has no sense of incongruity. If, however, beat noise-like amplitude variation component overlaps a frequency component of image information as shown in Fig. 7(3-b), the observer of the image recognizes this overlap as an artifact, and a stripe pattern gives the observer a sense of incongruity. The frequency of this amplitude variation is given by |fs/2 - fg|. When some nonlinear conversion is performed for the image, this variation component may actually become an artifact that impairs the image.

Ideally, all artifact frequencies should fall outside the frequency fi of the image. In this case, no problem arises. Although the frequency fi is the maximum frequency of the image, this frequency may be regarded as a frequency that gives the observer no sense of incongruity when the image is reproduced (hard copy, monitor display, or the like). Both inequality (3) and |fs/2 - fg| > fi must be satisfied at once. Assuming that fg is set at a position corresponding to fs > fg > fs/2, the following inequality is established:

$$fi + fs/2 < fg < fs - fi \qquad \ldots (4)$$

To satisfy this inequality, fi < fs/4 must also be satisfied, which is a strict condition. If fi < fs/4 is not satisfied in inequality (4), there is no overlap between the ranges defined by the left- and right-side signs. That is, an ideal condition for eliminating the influences of the grid is that the frequency band of the image is equal to or less than 1/2 the Nyquist frequency, and the frequency of the grid stripe pattern is near 1/2 the Nyquist frequency. This condition means that if an image to be acquired is determined, the sampling frequency must be set to at least four times higher than the maximum spatial frequency of the image to be acquired.

No consideration, however, is given to the power of beat noise-like variation component. The present inventor has contrived and proven that the condition given by inequality (4) can be moderated, on the basis of the result obtained by comparing the power of the beat noise-like variation component with the power of the grid stripe pattern and the result obtained by observing the image actually formed by using the grid.

As the frequency of the stripe pattern separates farther from the Nyquist frequency, the amplitude variation as beat noise increases in frequency. As a consequence, this component cannot be observed. Consider how much this stripe pattern frequency differs from the Nyquist frequency when it becomes difficult for the observer to observe the variation component.

Line spectra always exist at positions which have a mirror-image relationship centering on the Nyquist frequency whenever sampling is performed, and beat noise is always produced between them. In the cases shown in Figs. 7(1-b) and 7(2-b), although beat noise is produced, it does not exist apparently. One conceivable reason for this is attributed to frequency; in the case shown in Fig. 7(2-a) or 7(3-a), the distance (frequency) between the two spectra having a mirror-image relationship centering on the Nyquist frequency is large, and hence the beat noise frequency becomes high. At this time, the spectrum of the fundamental sine wave exists sufficiently below the Nyquist frequency, and they greatly differ in frequency and power. For this reason, only the fundamental sine wave having stronger power is strongly recognized by the observer.

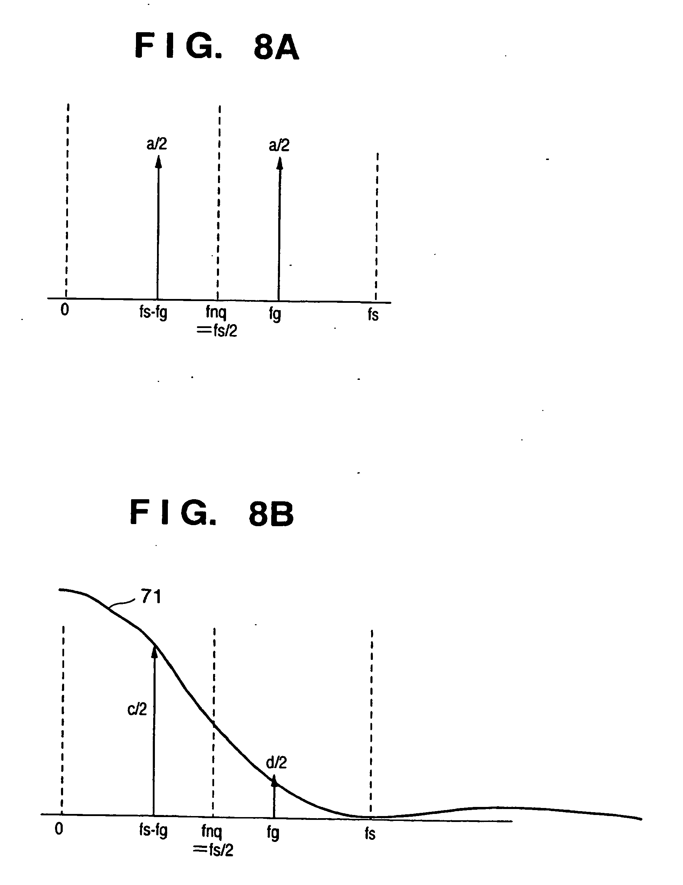

The manner in which beat noise is produced is mathematically expressed. Consider a case wherein a sine wave having the spatial frequency fg is sampled at the sampling frequency fs. Assume that fg > fs/2 is set in consideration of the grid actually used. This is not a necessary condition. In this case, as shown in Fig. 8A, a line spectrum pair corresponding to the frequency of the sine wave is produced. Let a/2 be the peak of each line spectrum, and a x cos $(2\pi fgx)$ be the initial grid image. When a cosine wave having the frequency fg is sampled at the frequency fs, two cosine waves appear below fs.

$$
\begin{aligned}
g(x) &= a\{\cos(2\pi fgx) \\
&\quad + \cos(2\pi(fs - fg)x)\} \\
&= 2a \times \cos(2\pi(fg - fs/2)x) \\
&\quad \times \cos(2\pi fsx/2) \qquad\qquad \ldots(5)
\end{aligned}
$$

Equation (5) represents beat noise, which is equivalent to the amplitude modulated by a sine wave having a frequency corresponding to the difference between the two sine waves. As (fg/2 - fs) becomes a small value other than 0, an unstable amplitude variation (beat) with a low frequency occurs.

According to the Shannon's sampling theorem, data sampled at a frequency equal to or lower than the Nyquist frequency can be completely reconstructed by using an ideal filtering means (a filter that passes signals having frequencies equal to or lower than the Nyquist frequency), and no beat is produced by the line spectrum pair. It is likely that the observer strongly recognizes the beat noise in the case shown in Fig. 7(3-b) because of the filtering means.

In general, a sampled signal is reconstructed by connecting the sampling points with a straight line or the like. This means differs from an ideal filtering means according to the sampling theorem.

A person with normal visual perception or display apparatus interpolates by connecting neighboring points with a straight line without using any ideal filter (convolution using a sinc function as a kernel) as in the sampling theorem. The same applies to a case wherein a signal is observed as an image. That is, such a difference between normal visual perception and the sampling theorem appears as beat noise.

Interpolating with a straight line amounts to filtering with a characteristic like a characteristic curve 71 in Fig. 8B.

According to the characteristic curve 71 in Fig. 8B, a form s(f) of the filter is given by

$$s(f) = \sin^2(\pi f/fs)/(\pi f/fs)^2 \qquad \ldots (6)$$

Let c/2 and d/2 be the heights of a line spectrum pair at mirror-image positions after filtering.
The sum of sine waves at this time is expressed like equation (5):

$$g(x) = d \times \cos(2\pi fgx) -$$
$$+ c \times \cos(2\pi(fs - fg)x)$$
$$= 2d \times \cos(2\pi(fg/2 - fs)x)$$
$$\times \cos(2\pi fsx/2)$$
$$+ (c - d) \times \cos(2\pi(fs$$
$$- fg)x) \qquad \ldots (7)$$

The first term of equation (7) represents beat component; and the second term, a general sine wave component.
According to equation (5), we have

$$c/2 = (a/2)\frac{\sin^2\left(\pi\dfrac{fs - fg}{fs}\right)}{\left(\pi\dfrac{fs - fg}{fs}\right)^2}, d/2 = (a/2)\frac{\sin^2\left(\pi\dfrac{fg}{fs}\right)}{\left(\pi\dfrac{fg}{fs}\right)^2}(fg > fs)$$

$$\ldots (8)$$

If in equation (7) the power of the second term which represents the normal sine wave component exceeds the power of the first term which represents the beat component, it may become difficult for the observer to recognize the beat component.
If the ratio of the power of the first term to that of the second term in equation (7) is calculated  on the basis of the above assumption, then
$(c - d)^2/2d^2 > 1$ (condition under which the power of the second terminal exceeds that of the first term)
This inequality can be rewritten into

$$c/d > 2^{1/2} + 1$$

A substitution of this into equation (7) yields

$$\frac{\dfrac{\sin^2\left(\pi\dfrac{fs-fg}{fs}\right)}{\left(\pi\dfrac{fs-fg}{fs}\right)^2}}{\dfrac{\sin^2\left(\pi\dfrac{fg}{fs}\right)}{\left(\pi\dfrac{fg}{fs}\right)^2}} > \sqrt{2}+1, \quad \frac{fg}{fs-fg} > \sqrt{\sqrt{2}+1}, \quad fg > \frac{fs}{\dfrac{1}{\sqrt{\sqrt{2}+1}}+1}$$

$$fg > 0.608\ fs$$

$$\ldots(9)$$

It is obvious from inequality (9) that if the frequency of a sine wave to be sampled is higher than 60.8% of a sampling frequency (aliasing occurs), sampling can be performed with little observable beat noise. In this case, grid stripe pattern information appears as a component equal to or less than 80% of the Nyquist frequency (fs/2). This component is equivalent to a component equal to or less than 40% of the sampling frequency. If, therefore, the grid stripe pattern has a frequency equal to or less than 80% of the Nyquist frequency, a stable stripe pattern can be observed without any conspicuous beat upon sampling.

The above consideration defines the upper limit spatial frequency of a stripe pattern (grid stripe pattern) appearing below the Nyquist frequency due to the grid. More specifically, the frequency of the grid stripe pattern is set to be equal to or less than 80% (equal to or less than 40% of the sampling frequency) of the Nyquist frequency. In practice, however, this frequency has its own lower limit. Since an artifact originating from the stripe pattern itself, i.e., stripe pattern information, is not allowed to overlap an image component, the maximum frequency of the image component needs to be lower than the lower limit frequency of the grid stripe pattern.

In general, the maximum frequency component of a signal representing an image cannot be accurately defined. Examples of evaluation criteria for images will be listed below:

- A frequency that meets the approval of the observer when an acquired image is reproduced by a display apparatus or recording apparatus.
- When a maximum frequency is assumed, the sampling pitch is determined by regarding 1.5 to 2 times the assumed frequency as a maximum frequency (see Nakamizo et al., " Counting/Measurement" (Baihukan)).

The latter condition is widely used; in particular, and the sampling pitch is often set such that a spatial frequency required generally is equal to or less than 60% of the Nyquist frequency (equal to or less than 30% of the sampling frequency). That is, the sampling pitch is set such that grid stripe pattern information appears at a frequency equal to or higher than the sampling frequency.

Assume that the sampling pitch is 0.1 mm (fs = 10 cyc/mm). In this case, the Nyquist frequency is 5 cyc/mm. According to the above general condition, the frequency of an image component which is generally used is equal to or less than 30% of the sampling frequency, i.e., 3 cyc/mm. That is, the lower limit frequency of stripe pattern information is 3 cyc/mm, and the upper limit frequency is 40% of the sampling frequency, i.e., 4 cyc/mm. If, therefore, the frequency of a grid stripe pattern is set within the range of 3 to 4 cyc/mm, no conspicuous beat appears in a grid stripe pattern and interferes with observation.

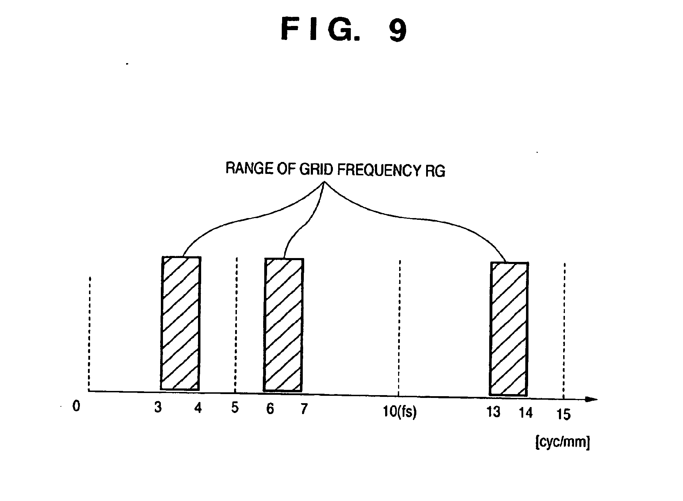

The above condition is set to determine the frequency of a grid stripe pattern, and the frequency of the lead members of the grid body whose stripe pattern corresponds to this value changes after sampling. In this case, as shown in Fig. 9 a frequency range RG [cyc/mm] of the grid body is given by

$$5(2n+1)-2 \sim 5(2n+1)-1 \text{ or } 5(2n+1)+1 \sim$$

$$(2n+1)+2; \ n = 0,\ 1,\ 2,\ldots \text{ (see Fig. 9)}$$

where n is an integer equal to or more than 0.

This mathematical expression represents a value calculated when the sampling pitch is 0.1 mm (fs = 10 cyc/mm). In general, if the sampling frequency fs [cyc/mm] is (sampling pitch 1/fs [mm]), the frequency range of the grid body is given by

$$\texttt{fs(n + 0.3) ~ fs(n + 0.4)}$$

or

$$\texttt{fs(n + 0.6) ~ fs(n + 0.8) [cyc/mm]}$$

The frequency of the grid body (the number of grid elements) is determined within the above range in consideration of scattered ray removing performance as the primary object of the grid, the resolution of a flat panel sensor, and the like. In general, in consideration of the resolution of the sensor, a grid stripe pattern frequency of 6 to 7 cyc/mm can be selected, at which the second harmonic is difficult to resolve and the scattered ray removing ratio is high.

In this example useful for understanding the invention, the frequency of the grid to be used is selected by the above calculation such that the observer can be satisfied to some degree without removing any grid stripe pattern information owing to some experience or depending on an application purpose, thus overcoming the problem.

As the spatial frequency of the grid stripe pattern is fixed, the grid stripe pattern information can be removed to some extent by filtering. In removing the grid stripe pattern information by setting a grid stripe pattern frequency in the above manner, even if the pattern information cannot be completely removed, reducing the intensity of the grid stripe pattern will minimize the influence on the observer.

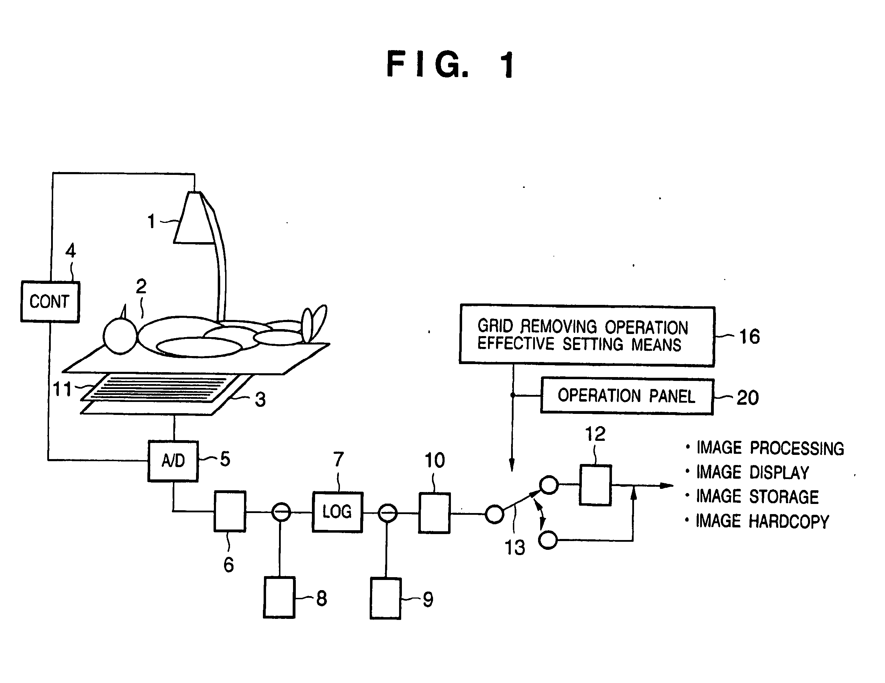

Fig. 1 is a schematic view of the first example useful for understanding the invention. Fig. 1 shows a system for imaging the human body lying on a table. Reference numeral 1 denotes an X-ray tube; 2, a human body as an object to be imaged; 11, a grid for removing scattered X-rays, which is a detachable grid for removing scattered X-rays from the object 2; 3, an X-ray sensor panel for converting an X-ray intensity distribution (X-ray transmission distribution) into a charge distribution, two-dimensionally sampling the distribution at desired intervals, and sequentially outputting the sampled data; 5, an analog/digital converter; 4, a controller for controlling the X-ray radiation timing and image acquisition timing; and 6, a memory for temporarily storing an image. The X-ray sensor panel 3 varies in offset and gain for each pixel. To correct this variation, an offset value as an image acquired without any radiation of X-rays is stored in a memory 8, whereas data obtained by logarithmically converting a gain value acquired without the object 2 and grid 11 is stored in a memory 9. Reference numeral 7 denotes a conversion unit for logarithmic conversion, and more specifically, a lookup table. An acquired image of the human body is logarithmically converted after the offset value in the memory 8 is subtracted (removed) from the image. The difference between the resultant value and the gain value in the memory 9 is calculated (division) to obtain an X-ray intensity distribution image having undergone correction of a variation in gain value. This image is temporarily stored in a memory 10. Thereafter, the stored image is extracted and subjected to image storage, image processing, image display, and hardcopy operation, and the like to be used for diagnosis and the like.

A block 12 is an image processing means (filtering means) for removing grid stripe pattern information by image processing (filtering). The image processing means 12 removes a grid stripe pattern component by spatial filtering using the image stored in the memory 10. A mechanism 13 is a selection means (switch) that is operated by the operator to choose between using the filtering means 12 or not using it in accordance with an output from an external output from a grid removing operation effective setting means (mechanism) 16 or operation panel 20. The flow of a signal can be changed to skip the operation of the block 12 in accordance with the selection made through the selection means 13.

In this case, the X-ray sensor panel 3 has a plurality of pixels distributed vertically and horizontally at a pitch of 0.1 mm in a two-dimensional space. With this structure, two-dimensional, discrete sampling is performed. As described in relation to the setting of the above grid stripe pattern frequency, the grid stripe pattern frequency (the number of grid elements) of the grid 11 is set to 6 to 7 cyc/mm, and hence the observer can observe an image without a sense of incongruity even if the grid tripe pattern information is not removed by filtering.

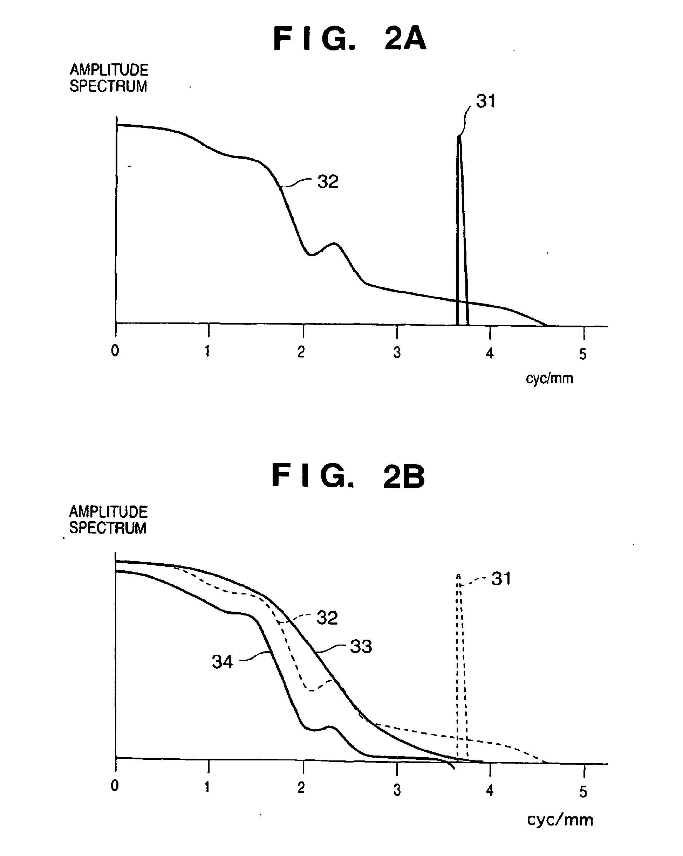

Fig. 2A schematically shows the state of a one-dimensional amplitude spectrum in a direction perpendicular to the grid stripe pattern of the image stored in the memory 10. Referring to Fig. 2A, reference numeral 32 denotes a spectrum of an image component; and 31, a spectrum of a grid stripe pattern component, which exhibits a substantial spectrum form with noise being neglected. Even if the grid stripe pattern component 31 exists, the observer observes only this stable frequency component, and there is no component associated with beat noise. Therefore, as the observer becomes accustomed to the stripe pattern or recognizes it, the existence of the stripe pattern does not relatively interfere with the observation.

If, however, the operator or observer wants to remove the grid stripe pattern information from this image owing to subsequent image processing or the image reproducing mechanism, he/she selects grid removing operation through the operation panel 20.

Fig. 2B schematically shows the state of a spectrum upon execution of grid removing filtering. Reference numeral 33 denotes an example of a filter characteristic; and 34, an image spectrum after filtering. To stabilize spatial characteristics, the filter cannot have a steep characteristic. If, therefore, the grid stripe pattern component 31 is removed, part of an image component inevitably deteriorates in response characteristic. In consideration of this, the operator chooses between removal and nonremoval of the grid. In this example useful for understanding the invention, when the acquired image exhibits the gain value stored in the memory 9, no problem arises if the image is acquired without removing the grid 11 because the frequency of the grid is constant.

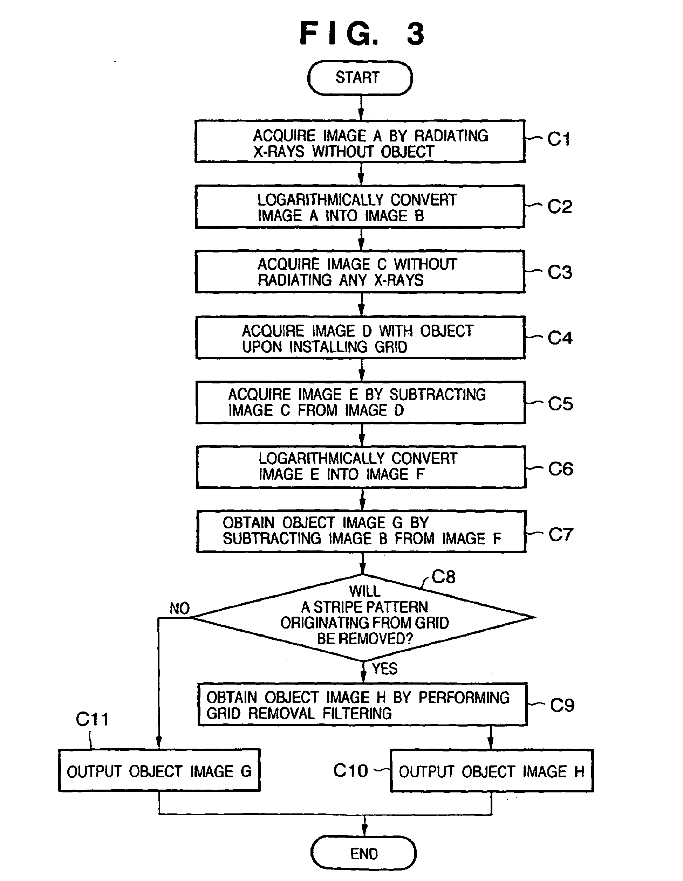

Fig. 3 is a flow chart for the implementation of this example useful for understanding the invention by means of software. Referring to Fig. 3, a process block (step) is divided into operations in blocks C1 to C11. The operation in block C1 is executed to acquire an image of a gain value. An image A is obtained by radiating X-rays without any object. In block C2, this image is logarithmically converted into an image B. In block C3, an offset value is obtained; an image C is obtained without radiating any X-rays. In block C4, an image of the object is actually acquired; the grid is installed, and an image D is obtained by irradiating the object with X-rays. In block C5, the image C is subtracted from the image D to obtain an image E having undergone offset correction. In block C6, the image E is logarithmically converted into an image F. In block C7, the image B is subtracted from the image F to obtain an object image G having undergone gain correction. In block C8, the flow branches depending on whether a stripe pattern operating from grid will be removed in accordance with operation (instruction) of the operation panel 20 by the operator. In block C9, since the instruction to remove the grid is received, filtering, i.e., grid removing operation, is performed for the image G to obtain an object image H from which grid stripe pattern information is removed. In block C10, the object image H is output. If no grid removing instruction is received, the object image G is output without any processing in block C11.

In this example useful for understanding the invention, as the memory means 10 in Fig. 1, a nonvolatile storage medium such as a magnetic disk may be used to always store image data including grid stripe pattern information to allow the operator to select an image without any grid stripe pattern information or an image with grid stripe pattern information or output them at once. Furthermore, this example useful for understanding the invention can be practiced with an arrangement including only an image storage system without any image acquisition system.

(Second example useful for understanding the invention)

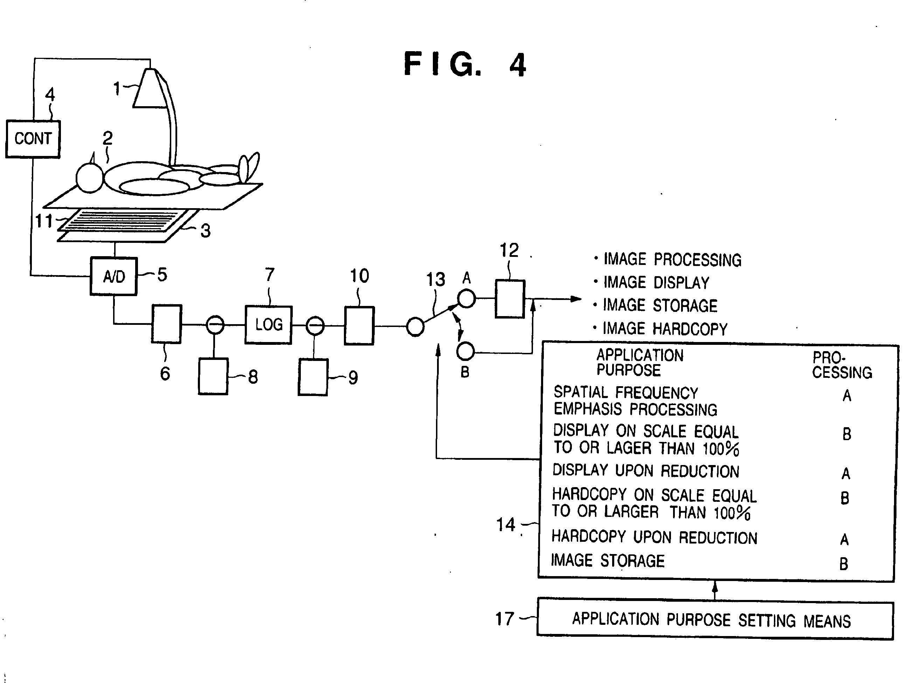

Fig. 4 is a block diagram showing the second example useful for understanding the invention, in which the frequency of a grid stripe pattern, the sampling pitch of an X-ray sensor panel, and the like are set in the same manner as in Fig. 1. In the structure shown in Fig. 4, an application purpose table 14 is prepared. After an image is acquired, the operator selects an application purpose through an application purpose setting means 17 with respect to the image stored in a memory 10 or magnetic disk. As a consequence, whether to remove the grid or not is automatically selected. If a switch 13 selects the A side, the image in the memory 10 is filtered by a filtering unit 12 and output. If the switch 13 selects the B side, the image in the memory 10 is output without being filtered.

As described in relation to the setting of the above grid stripe pattern frequency, the grid stripe pattern frequency (the number of grid elements) of a grid 11 is set to 6 to 7 cyc/mm, and hence the observer can observe an image without a sense of incongruity even if the grid tripe pattern information is not removed by filtering.

With this table 14, for example, in emphasizing a high spatial frequency as in spatial frequency emphasis processing, since a grid image becomes a hindrance, it is removed (the switch 13 is set on the A side). In displaying an image or performing hardcopy operation on a larger scale, i.e., 100% or more, the switch 13 is set on the B side to inhibit removal of a grid image so as to minimize an image blur. In displaying an image or performing hardcopy operation upon reduction, the switch 13 is set on the A side to remove a grid image. If an image is to be stored in another storage means, since removal processing can be performed for the stored image, the switch 13 is set on the B side to inhibit removal of a grid image, thus increasing the information amount.

(Third Embodiment)

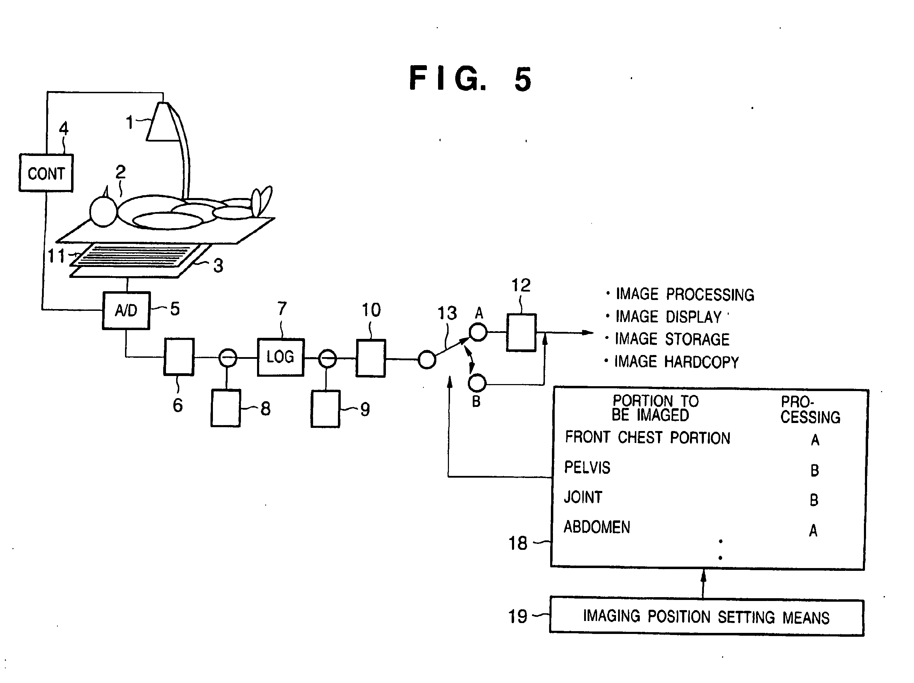

Fig. 5 is a block diagram showing the third embodiment, in which the frequency of a grid stripe pattern, the sampling pitch of an X-ray sensor panel, and the like are set in the same manner as in Fig. 1. In the structure shown in Fig. 5, an imaging position table 18 is prepared. When the operator selects an application purpose through an imaging position setting means 19 with respect to the image stored in a memory 10 or magnetic disk upon imaging operation, whether to perform grid removing operation or not is automatically selected.

As described in relation to the setting of the above grid stripe pattern frequency, the grid stripe pattern frequency (the number of grid elements) of a grid 11 is set to 6 to 7 cyc/mm, and hence the observer can observe an image without a sense of incongruity even if the grid tripe pattern information is not removed by filtering.

With this table 18, when the observer is to observe an image of a bone portion such as the pelvis or joint, which requires a high spatial frequency for the image, a switch 13 is set on the B side to allow the observer to observe the image without any blur without removing a grid image. When the observer is to observe a chest portion (front chest portion), abdomen, or the like for which a high spatial frequency is not required, and a halftone image needs to be easily observed, the switch 13 is set on the A side to allow the observer to observe the image without any grid image.

(Fourth example useful for understanding the invention)

Although the grid frequency is so set that a grid image does not relatively interfere with observation, if the contrast of the grid image is strong, it still interferes with the observer. The contrast of a grid image varies depending on the conditions (e.g., energy) of X-rays to be used. In some case, an object is imaged without any grid.

To determine this, the spectrum of a given portion of an acquired image in a direction perpendicular to the grid is calculated, and whether to perform grid removing operation or not is selected depending on the peak value of the grid component (directly corresponding to the contrast value if it is a logarithmic image).

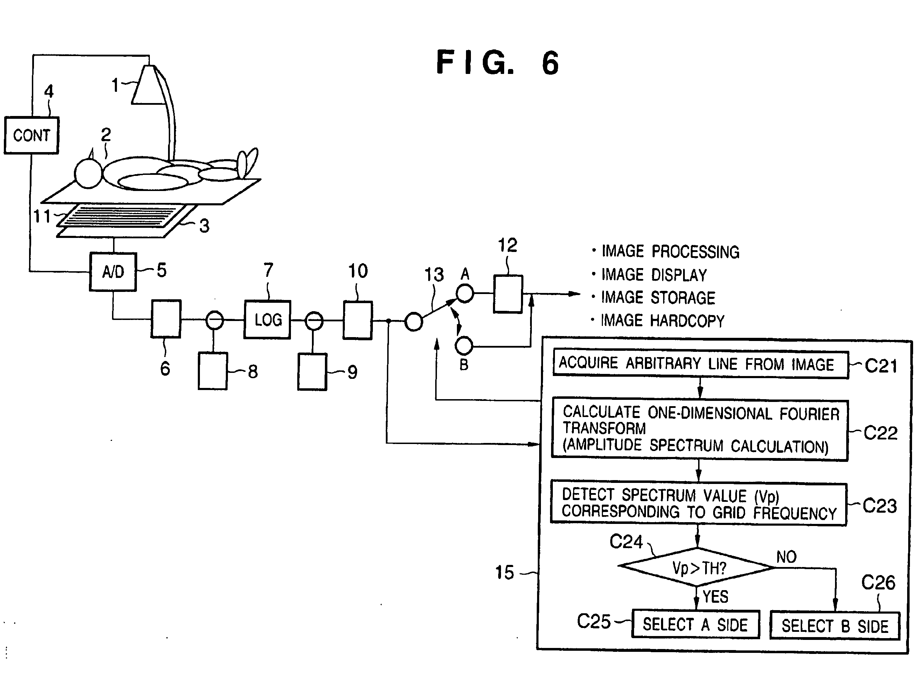

Fig. 6 is a block diagram showing the fourth example useful for understanding the invention, in which the frequency of a grid stripe pattern, the sampling pitch of an X-ray sensor panel, and the like are set in the same manner as in Fig. 1. As described in relation to the setting of the above grid stripe pattern frequency, the grid stripe pattern frequency (the number of grid elements) of a grid 11 is set to 6 to 7 cyc/mm, and hence the observer can observe an image without a sense of incongruity even if the grid stripe pattern information is not removed by filtering.

A block 15 in Fig. 6 includes software; a flow chart is shown in the block. In block C21 in the flow chart, an arbitrary line is acquired from an image from a memory 10. In block C22, one-dimensional Fourier transformation is performed for this image to calculate an amplitude spectrum. In block C23, an amplitude value (spectrum value) Vp corresponding to the grid frequency is measured from the amplitude spectrum value. In block C24, the amplitude value Vp is compared with a threshold TH set in advance. If Vp is larger than TH, the corresponding grid image must be removed. To remove the grid image, therefore, a switch 13 is set on the A side in block C25. If Vp is not larger than TH, the switch 13 is set on the B side in block C26.

Note that the switch 13 may be selectively operated in accordance with the magnitude of contrast of a grid stripe pattern existing at the grid frequency. If the contrast is higher than a predetermined threshold, the switch 13 is set on the A side to remove a grid image. If the contrast is lower than the predetermined threshold, the switch 13 is set on the B side to inhibit the removal of a grid image.

As described above, according to this example useful for understanding the invention, in a system for two-dimensionally sampling an X-ray image and forming a digital image, an image that can be observed by the observer with little sense of . incongruity without removing a grid stripe pattern from the image can be formed by setting the frequency of the grid lead members to be equal to or lower than 40% of the sampling frequency at which the spatial frequency of a stripe image originating from the grid for removing scattered rays from the image and equal to or higher than the frequency (60% of the Nyquist frequency in general) at which a grid image does not easily interfere with observation of the image by the observer and does not overlap an image component.

In addition, the filtering means 12 can choose between removal or nonremoval of a grid stripe pattern in accordance with selection through the switch 13. Since a grid stripe pattern is automatically removed in accordance with operation by the operator (observer), the application purpose of an image, imaging position, and the amplitude (intensity) of a grid stripe pattern, an appropriate image can be obtained by removing a grid stripe pattern only when required.

Each embodiment described above is merely an example in practicing the present invention. Note that the technical scope of the present invention should not be interpreted in a limited manner. That is, the present invention can be practiced in various forms without departing from the scope of the present invention.

As has been described above, according to each embodiment described above, when an X-ray image is two-dimensionally sampled to acquire a digital image, an image that can be observed by the observer with little sense of incongruity without removing a grid stripe pattern from the image can be formed by setting the spatial frequency of a stripe pattern originating from the grid for removing scattered rays from the image to a predetermined value.

In addition, since whether to remove a stripe pattern originating from the scattered ray removing grid from an image can be selected, an appropriate image can be obtained by removing a stripe pattern originating from the scattered ray removing grid.

The present invention is not limited to the above embodiments and various changes and modifications can be made within the scope of the present invention. Therefore, to apprise the public of the scope of the present invention the following claims are made.

It is an object of this invention to provide an image acquisition apparatus which can acquire an image which prevents a stripe pattern originating from a scattered ray removing grid from interfering with observation when X-rays are radiated by using the scattered ray removing grid. An image acquisition apparatus of an example useful for understanding the invention radiates X-rays by using a scattered ray removing grid (11) and two-dimensionally samples an X-ray transmission distribution on an object (2) to be imaged at desired sampling intervals. This apparatus includes an image acquisition means for acquiring an image by setting the intervals of elements of the scattered ray removing grid, without moving the scattered ray removing grid, such that the spatial frequency of a stripe pattern, in the image, which originates from the scattered ray removing grid becomes 40% or more of a sampling frequency that is the reciprocal of the sampling intervals.

**Claims**

1. An image acquisition apparatus comprising:

   image acquisition means (1, 3) for radiating radiation by using a scattered ray removing grid (11) and acquiring an image by two-dimensionally sampling a radiation transmission distribution on an object (2) to be imaged at sampling intervals;
   image processing means (12) for removing a stripe pattern originating from the scattered ray removing grid from the acquired image by image processing; and
   selection means (13) for allowing selection between removal and nonremoval of the stripe pattern originating from the scattered ray removing grid by using said image processing means,
   wherein said selection means is configured to select between removing the stripe pattern or not removing the stripe pattern in accordance with an imaging position selected in accordance with an operation performed by an operator, wherein the imaging position indicates a portion of the object to be imaged.

2. The apparatus according to claim 1, wherein said image processing means is configured to remove the stripe pattern originating from the scattered ray removing grid by filtering.

3. The apparatus according to any of claims 1 to 2, wherein said selection means is configured to select not removing the stripe pattern if the imaging position is a bone portion.

4. The apparatus according to any of claim 1 to 3, wherein said selection means is configured to select not removing the stripe pattern if the imaging position is a pelvis or joint.

5. The apparatus according to any of claims 1 to 4, wherein said selection means is configured to select removing the stripe pattern if the imaging position is a chest portion or abdominal portion.

6. The apparatus according to any of claims 1 to 5, further comprising offset value removing means (8) for removing an offset value from the image acquired by radiating the radiation.

7. The apparatus according to claim 6, wherein said offset value removing means (8) includes first subtraction means for subtracting an image acquired without radiating radiation from the image acquired by radiating the radiation.

8. The apparatus according to any of claims 1 to 7, further comprising gain value correction means (7, 9) for correcting a variation in gain value with respect to the acquired image.

9. The apparatus according to claim 8, wherein said gain value correction means includes division means configured to divide the acquired image by an image acquired without the object.

10. The apparatus according to claim 8, wherein said gain value correction means (7) includes second subtraction means for subtracting an image obtained by logarithmically converting an image acquired without the object from an image obtained by logarithmically converting the acquired image.

11. The apparatus according to any of claims 1 to 10, wherein
    said image acquisition means is further configured to acquire an image by setting intervals of elements of the scattered ray removing grid such that a spatial frequency of a stripe pattern, in the image, which originates from the scattered ray removing grid becomes less than 40% of a  sampling frequency that is the reciprocal of the sampling intervals, when the scattered ray removing grid is used without being moved.

12. The apparatus, according to claim 11, wherein

the spatial frequency becomes less than 30% of a sampling frequency that is the reciprocal of the sampling intervals.

**13.** The apparatus according to any of claims 1 to 12, wherein
said image acquisition means is configured to acquire an image by setting intervals of elements of the scattered ray removing grid such that a spatial frequency range of a stripe pattern, in the image, which originates from the scattered ray removing grid becomes fs(n + 0.3) ~ fs(n + 0.4) or fs(n + 0.6) ~ fs (n + 0.8) [cyc/mm] where 1/fs [mm] is the sampling intervals, fs is a positive number, and n is an integer not less than 0, wherein the scattered ray removing grid is adapted to be used without being moved.

**14.** An image acquisition method comprising:

image acquisition step of radiating radiation by using a scattered ray removing grid (11) and acquiring an image by two-dimensionally sampling a radiation transmission distribution on an object (2) to be imaged at sampling intervals;

image processing step of removing a stripe pattern originating from the scattered ray removing grid from the acquired image by image processing; and

selection step of allowing selection between removal and nonremoval of the stripe pattern originating from the scattered ray removing grid by using said image processing step,

wherein said selection step is configured to select between removing the stripe pattern or not removing the stripe pattern in accordance with an imaging position selected in accordance with an operation performed by an operator, wherein the imaging position indicates a portion of the object to be imaged.

**Patentansprüche**

**1.** Bilderhaltevorrichtung mit:

Bilderhalteeinrichtung (1, 3) zum Bestrahlen mit einer Strahlung unter Verwendung eines Streustrahlentfernungsgitters (11) und Erhalten eines Bildes durch zweidimensionales Abtasten einer Strahlungstransmissionsverteilung auf einem abzubildenden Objekt (2) mit Abtastabständen;

Bildverarbeitungseinrichtung (12) zum Entfernen eines Streifenmusters, das von dem Streustrahlentfernungsgitter herrührt, aus dem erhaltenen Bild durch Bildverarbeitung; und

Auswahleinrichtung (13), die eine Auswahl zwischen Entfernen und Nichtentfernen des Streifenmusters, das von dem Streustrahlentfernungsgitter herrührt, unter Verwendung der Bildverarbeitungseinrichtung ermöglicht, wobei die Auswahleinrichtung konfiguriert ist, zwischen Entfernen des Streifenmusters und Nichtentfernen des Streifenmusters gemäß einer Abbildungsposition auszuwählen, die gemäß einer durch einen Bediener ausgeführten Bedienvorgang ausgewählt wurde, wobei die Abbildungsposition einen Abschnitt des abzubildenden Objekts anzeigt.

**2.** Vorrichtung nach Anspruch 1, wobei die Bildverarbeitungseinrichtung konfiguriert ist, das Streifenmuster, das von dem Streustrahlentfernungsgitter herrührt, durch Filtern zu entfernen.

**3.** Vorrichtung nach einem der Ansprüche 1 bis 2, wobei die Auswahleinrichtung konfiguriert ist, das Streifenmuster nicht zu entfernen, wenn die Abbildungsposition ein Knochenabschnitt ist.

**4.** Vorrichtung nach einem der Ansprüche 1 bis 3, wobei die Auswahleinrichtung konfiguriert ist, Nichtentfernen des Streifenmusters auszuwählen, wenn die Abbildungsposition ein Becken oder ein Gelenk ist.

**5.** Vorrichtung nach einem der Ansprüche 1 bis 4, wobei die Auswahleinrichtung konfiguriert ist, Entfernen des Streifenmusters auszuwählen, wenn die Abbildungsposition ein Brustabschnitt oder ein abdominaler Abschnitt ist.

**6.** Vorrichtung nach einem der Ansprüche 1 bis 5, ferner mit einer Offsetwertentfernungseinrichtung (8) zum Entfernen eines Offsetwerts von dem Bild, das durch Bestrahlen mit der Strahlung erhalten wurde.

**7.** Vorrichtung nach Anspruch 6, wobei die Offsetwertentfernungseinrichtung (8) eine erste Subtraktionseinrichtung zum Subtrahieren eines Bildes, das ohne Bestrahlung mit der Strahlung erhalten wurde, von dem Bild, das durch Bestrahlen mit der Strahlung erhalten wurde, aufweist.

8. Vorrichtung nach einem der Ansprüche 1 bis 7, ferner mit einer Verstärkungswertkorrektureinrichtung (7, 9) zum Korrigieren einer Variation in einem Verstärkungswert mit Bezug auf das erhaltene Bild.

9. Vorrichtung nach Anspruch 8, wobei die Verstärkungswertkorrektureinrichtung eine Divisionseinrichtung enthält, die konfiguriert ist, das erhaltene Bild durch ein Bild zu teilen, das ohne das Objekt erhalten wurde.

10. Vorrichtung nach Anspruch 8, wobei die Verstärkungswertkorrektureinrichtung eine zweite Subtraktionseinrichtung (7) zum Subtrahieren eines Bildes, das durch logarithmisches Konvertieren eines Bildes, das ohne das Objekt erhalten wurde, von einem Bild, das durch logarithmisches Konvertieren des erhaltenen Bildes erhalten wurde, aufweist.

11. Vorrichtung nach einem der Ansprüche 1 bis 10, wobei die Bilderhalteeinrichtung ferner konfiguriert ist, ein Bild durch Setzen von Abständen von Elementen des Streustrahlentfernungsgitters so zu setzen, dass eine räumliche Frequenz eines Streifenmusters in dem Bild, die von dem Streustrahlentfernungsgitter herrührt, weniger als 40% einer Abtastfrequenz wird, die der Kehrwert der Abtastabstände ist, wenn das Streustrahlentfernungsgitter verwendet wird, ohne bewegt zu werden.

12. Vorrichtung nach Anspruch 11, wobei
die räumliche Frequenz weniger als 30% einer Abtastfrequenz wird, die der Kehrwert der Abtastabstände ist.

13. Vorrichtung nach einem der Ansprüche 1 bis 12, wobei
die Bilderhalteeinrichtung konfiguriert ist, ein Bild durch Setzen von Abständen von Elementen des Streustrahlentfernungsgitters so zu setzen, dass ein räumlicher Frequenzbereich eines Streifenmusters in dem Bild, das von dem Streustrahlentfernungsgitter herrührt,
$fs(n + 0,3) \sim fs(n + 0,4)$ oder $fs(n + 0,6) \sim fs(n + 0,8)$ [cyc/mm] wird, wobei $1/fs$ [mm] der Abtastabstand ist, $fs$ eine positive Zahl ist, und $n$ eine ganze Zahl nicht kleiner als 0 ist, wobei das Streustrahlentfernungsgitter angepasst ist, verwendet zu werden, ohne bewegt zu werden.

14. Bilderhalteverfahren mit:

einem Bilderhalteschritt des Bestrahlens mit Bestrahlung unter Verwendung eines Streustrahlentfernungsgitters (11) und Erhalten eines Bildes durch zweidimensionales Abtasten einer Strahlungstransmissionsverteilung auf einem abzubildenden Objekt (2) mit Abtastabständen;
einem Bildverarbeitungsschritt des Entfernens eines Streifenmusters, das von dem Streustrahlentfernungsgitter herrührt, aus dem erhaltenen Bild durch ein Bildverarbeiten; und
einem Auswahlschritt des Ermöglichens einer Auswahl zwischen Entfernen und Nichtentfernen des Streifenmusters, das von dem Streustrahlentfernungsgitter herrührt, unter Verwendung des Bildverarbeitungsschritts, wobei der Auswahlschritt konfiguriert ist, zwischen Entfernen des Streifenmusters oder Nichtentfernen des Streifenmusters gemäß einer Bildposition auszuwählen, die gemäß einem Bedienvorgang ausgewählt wurde, der durch einen Bediener durchgeführt wurde, wobei die Bildposition einen Abschnitt des abzubildenden Objekts anzeigt.

## Revendications

1. Appareil d'acquisition d'image comprenant :

un moyen (1, 3) d'acquisition d'image destiné à projeter un rayonnement en utilisant une grille (11) d'élimination de rayonnement diffus et à acquérir une image en échantillonnant de façon bidimensionnelle, à des intervalles d'échantillonnage, une distribution de transmission de rayonnement sur un objet (2) dont l'image est à former ;
un moyen (12) de traitement d'image destiné, par traitement d'image, à éliminer, de l'image acquise, un motif en forme de bandes ayant pour origine la grille d'élimination de rayonnement diffus ; et
un moyen (13) de sélection destiné à permettre une sélection entre l'élimination et la non-élimination du motif en bandes ayant pour origine la grille d'élimination de rayonnement diffus en utilisant ledit moyen de traitement d'image,
dans lequel ledit moyen de sélection est configuré pour sélectionner l'élimination du motif en bandes ou la non-élimination du motif en bandes en fonction d'une position de formation d'image sélectionnée d'après une opération effectuée par un opérateur, la position de formation d'image indiquant une partie de l'objet dont l'image est à former.

2. Appareil selon la revendication 1, dans lequel ledit moyen de traitement d'image est configuré pour éliminer par filtrage le motif en bandes ayant pour origine la grille d'élimination de rayonnement diffus.

3. Appareil selon l'une quelconque des revendications 1 et 2, dans lequel ledit moyen de sélection est configuré pour sélectionner la non-élimination du motif en bandes si la position de formation d'image est une partie osseuse.

4. Appareil selon l'une quelconque des revendications 1 à 3, dans lequel ledit moyen de sélection est configuré pour sélectionner la non-élimination du motif en bandes si la position de formation d'image est un bassin ou une articulation.

5. Appareil selon l'une quelconque des revendications 1 à 4, dans lequel ledit moyen de sélection est configuré pour sélectionner l'élimination du motif en bandes si la position de formation d'image est une partie de cage thoracique ou une partie abdominale.

6. Appareil selon l'une quelconque des revendications 1 à 5, comprenant en outre un moyen (8) d'élimination de valeur de décalage destiné à éliminer, de l'image acquise par projection du rayonnement, une valeur de décalage.

7. Appareil selon la revendication 6, dans lequel ledit moyen (8) d'élimination de valeur de décalage inclut un premier moyen de soustraction destiné à soustraire, de l'image acquise par projection du rayonnement, une image acquise sans projection de rayonnement.

8. Appareil selon l'une quelconque des revendications 1 à 7, comprenant en outre un moyen (7, 9) de correction de valeur de gain destiné à corriger la variation d'une valeur de gain en ce qui concerne l'image acquise.

9. Appareil selon la revendication 8, dans lequel ledit moyen de correction de valeur de gain inclut un moyen de division configuré pour diviser l'image acquise par une image acquise sans l'objet.

10. Appareil selon la revendication 8, dans lequel ledit moyen (7) de correction de valeur de gain inclut un second moyen de soustraction destiné à soustraire, d'une image obtenue en convertissant de façon logarithmique l'image acquise, une image obtenue en convertissant de façon logarithmique une image acquise sans l'objet.

11. Appareil selon l'une quelconque des revendications 1 à 10, dans lequel ledit moyen d'acquisition d'image est en outre configuré pour acquérir une image en réglant les intervalles d'éléments de la grille d'élimination de rayonnement diffus de façon que la fréquence spatiale d'un motif en bandes, dans l'image, qui a pour origine la grille d'élimination de rayonnement diffus devienne inférieure à 40 % de la fréquence d'échantillonnage qui est l'inverse des intervalles d'échantillonnage, lorsque la grille d'élimination de rayonnement diffus est utilisée sans la mouvoir.

12. Appareil selon la revendication 11, dans lequel la fréquence spatiale devient inférieure à 30 % de la fréquence d'échantillonnage qui est l'inverse des intervalles d'échantillonnage.

13. Appareil selon l'une quelconque des revendications 1 à 12, dans lequel ledit moyen d'acquisition d'image est configuré pour acquérir une image en réglant les intervalles des éléments de la grille d'élimination de rayonnement diffus de façon que la gamme de fréquences spatiales d'un motif en bandes, dans l'image, qui a pour origine la grille d'élimination de rayonnement diffus devienne fs(n + 0,3) - fs(n + 0,4) ou fs(n + 0,6) - fs(n + 0,8) [cyc/mm] où 1/fs [mm] est l'intervalle d'échantillonnage, fs est un nombre positif, et n est un nombre entier non inférieur à 0, dans lequel la grille d'élimination de rayonnement diffus est apte à être utilisée sans la mouvoir.

14. Procédé d'acquisition d'image comprenant :

une étape d'acquisition d'image consistant à projeter un rayonnement en utilisant une grille (11) d'élimination de rayonnement diffus et à acquérir une image en échantillonnant de façon bidimensionnelle, à des intervalles voulus, une distribution de transmission de rayonnement sur un objet (2) dont l'image est à former ;
une étape de traitement d'image consistant, par traitement d'image, à éliminer, de l'image acquise, un motif en forme de bandes ayant pour origine la grille d'élimination de rayonnement diffus ; et
une étape de sélection consistant à permettre une sélection entre l'élimination et la non-élimination du motif en bandes ayant pour origine la grille d'élimination de rayonnement diffus en utilisant ladite étape de traitement d'image,

dans lequel ladite étape de sélection est configurée pour sélectionner l'élimination du motif en bandes ou la non-élimination du motif en bandes en fonction d'une position de formation d'image sélectionnée d'après une opération effectuée par un opérateur, la position de formation d'image indiquant une partie de l'objet dont l'image est à former.

# F I G. 1

EP 1 126 289 B1

# F I G.  2A

AMPLITUDE
SPECTRUM

31

32

0    1    2    3    4    5

cyc/mm

# F I G.  2B

AMPLITUDE
SPECTRUM

31

32

33

34

0    1    2    3    4    5

cyc/mm

# F I G. 3

START

ACQUIRE IMAGE A BY RADIATING X-RAYS WITHOUT OBJECT — C1

LOGARITHMICALLY CONVERT IMAGE A INTO IMAGE B — C2

ACQUIRE IMAGE C WITHOUT RADIATING ANY X-RAYS — C3

ACQUIRE IMAGE D WITH OBJECT UPON INSTALLING GRID — C4

ACQUIRE IMAGE E BY SUBTRACTING IMAGE C FROM IMAGE D — C5

LOGARITHMICALLY CONVERT IMAGE E INTO IMAGE F — C6

OBTAIN OBJECT IMAGE G BY SUBTRACTING IMAGE B FROM IMAGE F — C7

C8 — WILL A STRIPE PATTERN ORIGINATING FROM GRID BE REMOVED?

NO

YES

OBTAIN OBJECT IMAGE H BY PERFORMING GRID REMOVAL FILTERING — C9

C11 — OUTPUT OBJECT IMAGE G

C10 — OUTPUT OBJECT IMAGE H

END

# FIG. 4

- IMAGE PROCESSING
- IMAGE DISPLAY
- IMAGE STORAGE
- IMAGE HARDCOPY

| APPLICATION PURPOSE | PRO-CESSING |
|---|---|
| SPATIAL FREQUENCY EMPHASIS PROCESSING | A |
| DISPLAY ON SCALE EQUAL TO OR LAGER THAN 100% | B |
| DISPLAY UPON REDUCTION | A |
| HARDCOPY ON SCALE EQUAL TO OR LARGER THAN 100% | B |
| HARDCOPY UPON REDUCTION | A |
| IMAGE STORAGE | B |

APPLICATION PURPOSE SETTING MEANS

# FIG. 5

- IMAGE PROCESSING
- IMAGE DISPLAY
- IMAGE STORAGE
- IMAGE HARDCOPY

| PORTION TO BE IMAGED | PRO-CESSING |
|---|---|
| FRONT CHEST PORTION | A |
| PELVIS | B |
| JOINT | B |
| ABDOMEN | A |
| . | |
| . | |

IMAGING POSITION SETTING MEANS

EP 1 126 289 B1

# FIG. 6

EP 1 126 289 B1

- IMAGE PROCESSING
- IMAGE DISPLAY
- IMAGE STORAGE
- IMAGE HARDCOPY

ACQUIRE ARBITRARY LINE FROM IMAGE — C21

CALCULATE ONE-DIMENSIONAL FOURIER TRANSFORM (AMPLITUDE SPECTRUM CALCULATION) — C22

DETECT SPECTRUM VALUE (Vp) CORRESPONDING TO GRID FREQUENCY — C23

C24 — $Vp > TH?$ — NO

YES

C25 — SELECT A SIDE

C26 — SELECT B SIDE

# FIG. 7

# F I G. 8A

a/2

a/2

0        fs-fg        fnq        fg        fs
                      =fs/2

# F I G. 8B

71

c/2

d/2

0        fs-fg        fnq        fg        fs
                      =fs/2

# FIG. 9

RANGE OF GRID FREQUENCY RG

0     3  4  5  6  7      10(fs)     13  14  15

[cyc/mm]

# F I G. 10

81

82

83

84

85

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2507659 B
- JP 2754068 B
- JP 8088765 A
- JP 9075332 A
- JP 9078970 A
- US 5801385 A
- US 5050198 A
- US 6201249 B1
- JP 9283606 A
- US 4317179 A